# EUROPEAN PATENT APPLICATION

(11) **EP 0 904 729 A1**
(43) Date of publication of application: **31.03.1999**
(21) Application number: 98115371.1
(22) Date of filing: 14.08.1998
(51) Int. Cl.: A61B 5/083, G01N 33/497

(54) **Method for determining the concentration of NO in a breathing gas and an analyser for carrying out the method**

(30) Priority: 30.09.1997 SE 9703545
(71) Applicant: Siemens-Elema AB, 171 95 Solna (SE)
(72) Inventor: Olsson, Sven-Gunnar, 232 00 Arlöv (SE); Rydgren, Göran, 230 44 Bunkeflostrand (SE); Linge, Anders, 244 36 Kävlinge (SE)

(57) **Abstract**

A method and an analyser for determining the concentration of NO in a breathing gas, also containing O₂ with which NO reacts to form NO₂, are described. The problem in determining the NO content of a breathing gas in a reliable fashion is solved by, in a first stage, increasing the pressure of the breathing gas for a predefined period of time so NO present in the breathing gas is almost completely converted into NO₂, whereupon the concentration of NO₂ is measured and the concentration of NO is determined from the measured concentration of NO₂. An analyser (10) for carrying out the method comprises a first reciprocating pump (30) and a second reciprocating pump (36) which interact in compressing a sample of the breathing gas for a predefined period of time. The gas sample is then sent to a measurement chamber (46) in which the concentration of NO₂ is measured. An analysis unit (52) controls the entire analyzer (10) and determines the NO concentration from the measured concentration of NO₂.

## Description

The present invention relates to a method according to the preamble to claim 1.

The present invention also relates to an analyser according to the preamble to claim 5.

In recent years, the gas NO has attracted great interest in different kinds of therapy. A number of beneficial effects have been recorded when small doses of NO have been administered to a patient via the airways. For example, NO has been found to have a relaxing effect on smooth muscle, thereby improving oxygenation and reducing blood pressure across the lung. More comprehensive descriptions of the effects NO has hitherto displayed are provided in e.g. WO 92/10228 and US-A-5,427,797.

NO, usually diluted with N₂ in gas cylinders, is then mixed with a breathing gas, commonly a mixture of air and oxygen (O₂), before the final mixture is delivered to the patient. Examples of known mixing systems are described in EP-A-O 659 445 and SE-C-502 724.

The biggest problem with NO is that it is a highly reactive gas, which together with O₂ forms NO₂, a gas that is highly toxic even in small concentrations. Since breathing gas generally contains an elevated level of O₂, e.g. 50-80% O₂, special measures may be necessary to minimise the amount of NO₂ supplied to the patient.

Determining the patient's NO uptake is also a matter of interest in therapy with NO. In principle, this is performed by first determining the NO content of the breathing gas before it is supplied to the patient and then determining the NO concentration of the gas leaving the patient in expiration. The difference in NO concentration corresponds to uptake in the patient's body.

However, NO is not a very easy gas to measure. And, as noted above, NO is converted into NO₂ when it comes into contact with O₂, thereby affecting determination of the patient's uptake of NO. Known methods for measuring NO use e.g. chemoluminescence detectors, but they are expensive and sensitive to environmental parameters, such as moisture and the oxygen level in particular. So gas for analysis must have a specific moisture content for reliable measurements of the gas.

As regards the concentration of NO in breathing gas, the addition of NO to the breathing gas can be performed in any suitable way described in the cited references to the prior art in order to minimise conversion of NO before it reaches the patient. The NO concentration of inspired gas can accordingly be determined with relatively great accuracy. As regards expired gas, however, the problem in determining the NO concentration is greater. More time will have elapsed, by then and a larger percentage of NO will have been converted into NO₂.

One objective of the present invention is therefore to achieve a method for accurate determination of the concentration of NO in a breathing gas, preferably the concentration in expired breathing gas.

Another objective of the invention is to achieve an analyser for determining the concentration of NO in a breathing gas.

One such method is achieved in accordance with the invention in that the pressure of the breathing gas is increased for a predefined period of time, the end concentration of NO₂, when any NO present in the breathing gas has been almost completely converted into NO₂, is determined and the concentration of NO is calculated from the measured concentration of NO₂.

Instead of attempting to measure NO content directly and compensate the measurement or determination for the formation of NO₂ (the time-related equation for translating NO into NO₂ is known), the conversion of NO is accelerated, and the end concentration of NO₂ is determined instead. The level of NO can then be established from the end concentration of NO₂.

The term 'end concentration' refers to the concentration prevailing when (virtually all) the NO has been converted into NO₂. There are two advantageous ways of determining the end concentration. In one, the increase in pressure is set so high that NO is almost completely converted into NO₂. In the other, the concentration of NO₂ is measured at least twice during the increase in pressure so the conversion curve can be determined. The end concentration can then be calculated from the measured values and conversion curve determined.

When NO is completely converted into NO₂ the concentration of NO can be determined from the concentration of NO₂. The increased pressure accelerates the reaction between NO and O₂, because the partial pressure of the gases accordingly increases. This reaction varies with the cube of the pressure, so a doubling of the pressure increases conversion 8-fold, a tripling of the pressure increases conversion 27-fold etc. Since the temperature has some effect on these processes, the temperature should be kept constant or at least be known.

When absorption measurements are employed, it may then be advantageous to measure the concentration at the higher pressure, since molecular density and, accordingly, absorption are greater than at lower pressures.

A greater increase in pressure also means that a shorter variable period of time can be selected. Since pressure influences the rate of reaction in this way, this method is much safer than direct measurement of the NO concentration and compensation of this measurement with a conversion factor for the time the gas flowed through the system of lines after being mixed. This is because the system's gas pressure varies during inspiration and expiration, so the rate of reaction would accordingly vary as well. Taking both pressure variations and time into account in determinations of a compensation factor for converted NO would be particularly complicated.

If the existing concentration of NO in the breathing gas (i.e. the concentration of un-converted NO) is to be determined, the concentration of NO₂ can be measured before the pressure is increased in the breathing gas in order to convert NO into NO₂, and the actual concentration of NO can be determined from the NO₂ concentrations determined before and after the increase in the pressure of the breathing gas.

An analyser is achieved in accordance with the invention in that the analyser comprises the features set forth in the characterising part to claim 5.

In principle, the analyser comprises the components needed for carrying out the method, i.e. an analysis unit for determining the concentration of NO₂ and at least one pressurising means for increasing the pressure of the gas for a predefined period of time.

The pressurising means in particular can be devised in a number of different ways. Thus, the pressurising means can be part of a sampling system for extracting gas samples and consist of one or a plurality of pumps, connected in series, which successively compress the gas in one or more stages.

Alternately, the measurement chamber, in which the analysis is performed, can be designed so its volume can be reduced, thereby compressing the gas sample in the measurement chamber.

Advantageous embodiment of the analyser will be evident from the dependent claims to claim 5.

Embodiments of the method and analyser are described below in greater detail, referring to the figures in which
FIG. 1 shows a ventilator system with an analyser according to the invention;
FIG. 2 shows a first embodiment of the analyser;
FIG. 3 shows a second embodiment of the analyser; and
FIG. 4 shows a third embodiment of the analyser.

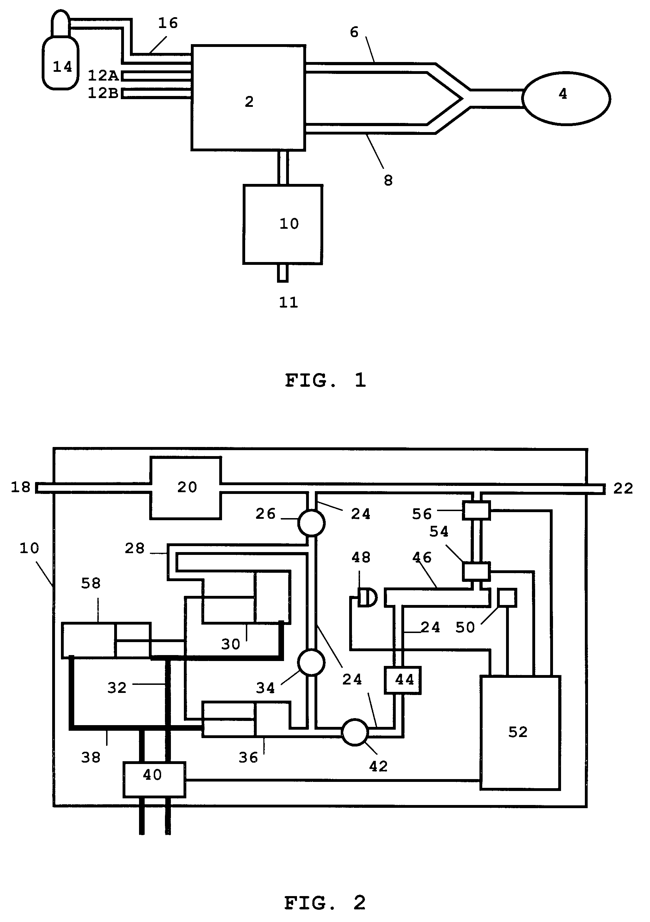

FIG. 1 shows a ventilator 2 connected to a patient 4 in order to supply a breathing gas, via an inspiratory line 6, to the patient 4 and carry expired gas, via an expiratory line 8, away from the patient 4 to an analysis unit 10 and an evacuation unit 11. The component gases in the breathing gas are supplied to the ventilator 2 via a first gas connection 12A for e.g. air and a second gas connector 12B for e.g. O₂. A therapeutic gas, NO in this instance, is supplied to the ventilator 2 from a gas cylinder 14, containing a mixture of e.g. 1000 ppm NO diluted with N₂, via a third gas connection 16.

The analyser 10 is connected between the ventilator 2 and the evacuation unit 11 in order to determine the NO content of expired breathing gas. When the NO content of inspired breathing gas is known, the uptake of NO in the patient 4 can be determined.

FIG. 2 shows a first embodiment of the analyser 10. Expired breathing gas is fed into the analyser 10 via a gas inlet 18 and carried to a mixing chamber 20. The purpose of the mixing chamber 20 is to achieve a homogenous composition for the breathing gas before it is measured. An average value for the NO concentration can then be determined, as well as an average value, or minute-volume, for the uptake of NO in the patient. Gas, which has been analysed, or gas which is not to be analysed, is evacuated from the analyser 10 via a gas outlet 22.

Gas samples to be analysed can be carried, via a gas sampling line 24, through a first check valve 26 and, via a gas line 28, to a first reciprocating pump 30 in which the gas sample is allowed to fill a space with a specific volume. When the gas sample fills the first reciprocating pump 30, the gas is compressed by the application of positive pressure via a first control gas line 32. The gas sample is forced out through the gas line 28 back to the gas sampling line 24, past a second check valve 34, and on to a second reciprocating pump 36. The volume of the second reciprocating pump 36 is much smaller than the volume of the first reciprocating pump 30 (e.g. a third or tenth as large), so the gas sample is compressed when it fills the second reciprocating pump 36. The ratio between the volumes is selected according to the pressure increase desired and the positive pressure available in the first control gas line 32. A large difference in volume causes a large pressure differential and, thus, more rapid conversion of NO into NO₂.

The second reciprocal pump 36 is also controlled by positive pressure gas applied via a second control gas line 38.

Application of positive pressure gas to the first control gas line 32 and to the second control gas line 38 is regulated via a switch 40. When the second reciprocating pump 36 is compressed, the gas sample is further compressed and forced back into the gas sampling line 24 in which the gas sample passes a third check valve 42, a catalyst and dehumidifier 44 before filling a measurement chamber 46. Catalysis further accelerates conversion and can help make conversion as complete as possible.

Compression of the gas sample, which also occurs for a specific period of time, increases the rate of reaction in the chemical reaction of NO and O₂, and virtually all the NO in the gas sample is converted into NO₂.

In the measurement chamber 46, the gas sample is illuminated by light from a light source 48, and the intensity of transilluminated light is sensed by a detector 50. The measurement signal generated is sent to an analysis unit 52, which also controls the light source 48 and switch 40. The analysis unit 52 determines the concentration of NO₂ gas in the specimen by means of traditional spectrophotometry. A reference signal for the intensity of radiated light can be obtained in the known manner, e.g. by the use of a reference chamber which does not contain NO₂, or even by measurement at a reference frequency at which NO₂ does not absorb radiation. To increase analysis precision, pressure in the measurement chamber 46 is measured by a pressure gauge 54, which sends the measurement signal to the analysis unit 52. The gas sample can then be carried to a gas outlet 22 through a valve 56, controlled by the analysis unit 52.

A third reciprocating pump 58 is mechanically connected to the first reciprocating pump 30 and to the second reciprocating pump 36 in order to control the pumping action of these pumps and even increase the compression capability.

At one end, the third reciprocating pump 58 is connected to the first control gas line 32 and, at the other end, to the second control gas line 38. The pistons of the three reciprocating pumps 30, 36, 58 are mechanically interlinked for efficient interaction.

Actual measurement of the NO₂ concentration can be performed with any other known measurement technique, in addition to conventional spectrophotometry.

When a valve (not shown) opening onto atmosphere is connected, ambient air can be brought into the analyser and used as a reference gas for calibration.

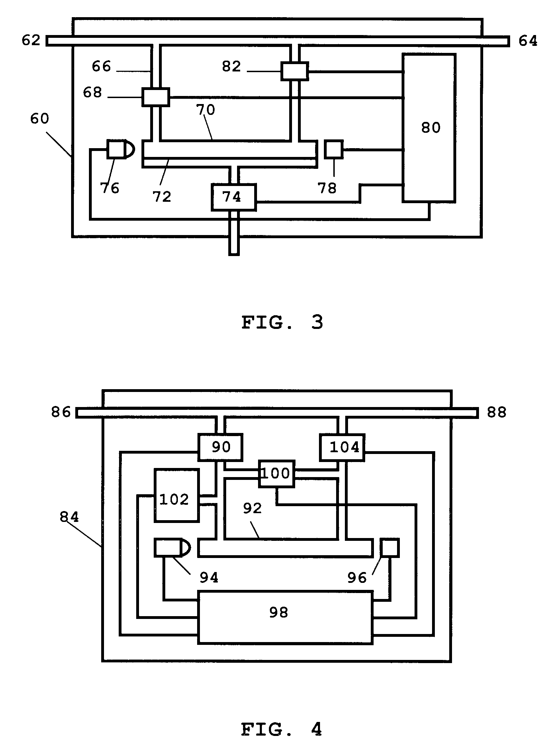

A second embodiment of the analyser is shown in FIG. 3 and is designated 60. Breathing gas is sent to the analyser 60 via a gas inlet 62. There is no mixing of the breathing gas in this instance. The breathing gas that is not to be analysed can pass straight to a gas outlet 64. The gas sample can be carried via a first valve 68 and a gas sampling line 66 to a measurement chamber 70.

The measurement chamber 70 is devised with a moving partition 72, which divides the measurement chamber 70 into two separate compartments. The partition 72 can be moved with compressed air, controlled by a control valve 74, to reduce the volume of that part of the measurement chamber 70 holding the gas sample. The gas is accordingly compressed, and any NO present in the gas sample is rapidly converted into NO₂. Compression is maintained for a specific period of time. Measurement can then be made by illuminating the gas sample with a light source 76 and sensing the intensity of the transilluminated light with a detector 78.

The measurement can either be made during or after the specific period of time. When measurements are made during the pressurisation period, the rate of conversion can be determined or, more accurately, the conversion curve can be determined for the prevailing pressure. On the basis thereof, the end concentration of NO₂ in the gas sample can be determined before all NO has been converted into NO₂. This speeds up determinations, especially in situations where the pressure cannot be increased very much.

When measurements are made after the specific period of time, a simple concentration measurement is enough. This can be done while the gas sample still has a positive pressure or after the pressure has been reduced to normal pressure. Measurement of concentration of NO₂ is, however, simpler at the elevated pressure.

After analysis, the gas sample is sent to the gas outlet 64 via a second valve 82.

If the content of NO, not yet converted into NO₂, in expired breathing gas is to be determined, the NO₂ content is measured before pressurisation.

Mechanical or hydraulic devices can be used, instead of compressed air, for moving the partition 72. The most important thing is to achieve a large, relatively rapid increase in pressure so as to accelerate the rate of reaction in the conversion of NO.

An analysis unit 80 regulates the valves 68, 82 in order to admit/discharge gas samples to/from the measurement chamber 70. It also controls the control valve 74. The analysis unit 80 additionally determines the NO concentration from the measured concentration of NO₂ before and after compression.

FIG. 4 shows a third embodiment of the analyser, designated 84. Breathing gas passes through the analyser 84 between a gas inlet 86 and a gas outlet 88. A gas sample can be taken from the breathing gas via a first valve 90 and carried straight into a measurement chamber 92 for analysis without any pressurisation, i.e. the NO₂ concentration is measured before NO has been completely converted. As in previous embodiments, a light source 94 and a detector 96 are employed for generating a measurement signal, which is analysed in an analysis unit 98. The analysis unit 98 also controls the entire analyser 84.

When the analysis has been completed, the gas sample is discharged from the measurement chamber 92 and carried, via a second valve 100, to a pressurising means 102 which can consist of a pressure chamber or the like. The gas sample is raised to a high pressure in the pressurising means 102 for a specific period of time in which virtually all the NO is converted into NO₂. The gas specimen is then returned to the measurement chamber 92, and the measurement is repeated. The difference in measurement results can be converted into the NO concentration the gas sample had when it was taken. This measurement method also supplies some information on how much NO₂ had time to form before the gas sample was taken.

The gas sample can now be sent to the gas outlet 88 via a third valve 104. All the valves 90, 100, 104 are controlled by the analysis unit.

Alternately, a first gas sample can be taken for measurement without any increase in pressure. A second gas sample is then taken and measured after pressurisation. This can be performed with all the embodiments.

Depending on the choice of wavelength in absorption measurement, compensation can be made, when necessary, for the N₂O₄ present in equilibrium with NO₂. The equilibrium ratio for N₂O₄ and NO₂ is well known, and a correction can be calculated by the analysis unit or stored in same. The ratio between the content of NO₂ and N₂O₄ is governed by the temperature. Determination of the temperature (or regulation of the temperature at the desired level) therefore provides an opportunity for compensating for this factor.

The various embodiments can, when appropriate, be intercombined. For example, a mixing chamber can be used in the second and third embodiments. In the corresponding manner, the mixing chamber 20 in the first embodiment can be omitted. The analyser 10 can also be located in the expiratory line 8 instead of after the ventilator 2 or even be incorporated into the ventilator 2. The pressurisation methods can also be combined. Thus, the successive increases in pressure produced by the pistons in the first embodiment can be combined with a pressurisation chamber according to the second embodiment for achieving the highest pressure increase possible. The pistons can then be driven by compressed air for a pressure increase up to about 5 bars of positive pressure. The pressurisation chamber can additionally increase the pressure by hydraulic means to e.g. about 10 bars of positive pressure. Conversion will then take place 1000 times more rapidly.

## Claims

1. A method for determining the concentration of NO in a breathing gas, also containing O₂ with which NO reacts to form NO₂, **characterised** in that the pressure of the breathing gas is increased over a variable period of time, the end concentration of NO₂, when the NO present in the breathing gas has been almost completely converted into NO₂, is determined and the concentration of NO is calculated from the determined concentration of NO₂.

2. The method according to claim 1, **characterised** in that the magnitude of the pressure increase is selected so conversion of NO into NO₂ is virtually complete during the variable period of time, and the end concentration of NO₂ is determined by measuring the concentration of NO₂, preferably after the variable period of time has lapsed.

3. The method according to claim 1, **characterised** in that the end concentration of NO₂ is determined by measuring the concentration of NO₂ at least twice during the variable period of time, the curve for conversion of NO into NO₂ is determined and the end concentration of NO₂ is established from the measured concentration of NO₂ and the determined conversion curve.

4. The method according to any of the above claims, **characterised** in that the concentration of NO₂ in breathing gas is also measured before the pressure of the breathing gas is increased, and the concentration of NO is determined from the determined concentrations of NO₂ before and after the increase in the pressure of the breathing gas.

5. An analyser (10; 60; 84) for determining the concentration of NO in a breathing gas, also containing O₂ with which NO reacts to form NO₂, comprising a measurement chamber (46; 70; 92), **characterised** by an analysis unit (48, 50, 52; 76, 78, 80; 94, 96 98) for measuring the concentration of NO₂ in the breathing gas and at least one pressurising means (30, 36; 72, 74; 102) devised to increase the pressure of the breathing gas over a predefined period of time, the analysis unit (48, 50, 50, 52; 76, 78, 80; 94, 96, 98) is devised to determine the end concentration of NO₂ in the breathing gas and the concentration of NO from the determined end concentration of NO₂.

6. The analyser according to claim 5, **characterised** by a sampling system (24-42; 68; 90, 100-104) for extracting a gas sample from the breathing gas and carrying it to the measurement chamber (46; 70; 92).

7. The analyser according to claim 6, **characterised** in that the pressurising means (30, 36: 102) is devised as a part of the sampling system (26-42; 90, 100-104) and preferably comprises at least one reciprocating pump (30, 36) devised to compress the gas sample.

8. The analyser according to claims 5 or 6, **characterised** in that the pressurising means (72, 74) is devised as a part of the measurement chamber (70).

9. The analyzer according to any of claims 5-8, **characterised** in that the analyser (84) is devised to measure the concentration of NO₂ in the breathing gas before the pressurising means (102) increases the pressure of the breathing gas, and the analysis unit (98) is devised to determine the concentration of NO from the concentrations of NO₂ determined before and after the pressure increase respectively.

10. The analyser according to claim 9 and 6, **characterised** in that the sampling system (90, 100-104), in a first position, is devised to carry a gas sample to the measurement chamber (92) for measurement of the concentration of NO₂ and, in a second position, to return the gas sample, via the pressurising means (102), for determination of the end concentration of NO₂.

11. The analyser according to claims 9 and 6, **characterised** in that the sampling system (68, 82; 90, 100-104), in a first position, is devised to carry a first gas sample to the measurement chamber (70; 90) for measurement of the concentration of NO₂ and, in a second position, to carry a second gas sample to the measurement chamber (70; 92), via the pressurising means (72, 74; 102), for determination of the end concentration of NO₂.

12. The analyser according to any of claims 5-11, **characterised** in that the analysis unit is devised to compensate determination of the concentration of NO for the presence of N₂O₄.
